# EUROPEAN PATENT APPLICATION

(11) **EP 4 570 172 A1**
(43) Date of publication of application: **18.06.2025**
(21) Application number: 23217196.7
(22) Date of filing: 15.12.2023
(51) Int. Cl.: A61B 5/0536, A61B 5/145

(54) **METHOD AND APPARATUS FOR ESTIMATING A CONCENTRATION OF A BLOOD ANALYTE**

(71) Applicant: CSEM Centre Suisse d'Electronique et de Microtechnique SA - Recherche et Développement, 2002 Neuchâtel (CH)
(72) Inventor: Chételat, Olivier, 1588 Cudrefin (CH)
(74) Representative: P&TS SA (AG, Ltd.)

(57) **Abstract**

The present disclosure concerns a method for determining the concentration of a blood analyte of a user, comprising: providing an apparatus (10) configured to acquire electrical impedance tomography (EIT) images within a measurement region of the thorax of the user, each EIT image comprising a plurality of EIT pixels, each of said EIT pixels comprising at least a first and second impedance values (Z[f1], Z[f2]) corresponding, respectively to a first and second measurement frequencies (f1, f2); using the apparatus (10) to measure a time-sequence of EIT images of said measurement region during a predetermined measuring time covering at least one heart cycle; determining a region of interest (ROI) within the measurement region, said ROI comprising at least a section of the lungs; and calculating the blood analyte concentration based on the impedance values of the EIT pixels within said ROI, from said time-sequence of EIT images. The present disclosure further concerns the apparatus (10) performing the method.

## Description

### Field

The present disclosure relates to a method and device for in-vivo and non-invasive estimation of blood analyte concentration in a user. More particularly, the present disclosure provides a method and device for estimating blood analyte concentration using electrical impedance tomography (EIT). The present disclosure further concerns an apparatus for performing the method.

### Background

Reference: Shokrekhodaei M., Quinones S. (2020) "Review of Non-invasive Glucose Sensing Techniques: Optical, Electrical and Breath Acetone", doi:10.3390/s20051251, reviews non-invasive techniques for measuring glucose concentration in blood, including techniques based on bioimpedance spectroscopy. Several challenges to this type of measurement are discussed in the paper; among them, the variability of tissue composition between individuals, motion artifacts, fluctuations of humidity and temperature at the skin where the electrodes are applied. Several works are cited, where the effect of temperature is dealt with by the introduction of empiric correction parameters in the calculation of glucose concentration.

Document US 2011028803 A1 discloses methods for estimating the glucose concentration in the blood of a subject, based on the simultaneous measurement of electric impedance and at least one concentration of an ion, typically acidity (pH), in a tissue of the subject, typically at the skin. As in other similar approaches, this technique suffers from the limitations mentioned in the review paper of Shokrekhodaei et al., related to tissue heterogeneity, motion artifacts, humidity, and temperature effects. This document mentions accessorily the possibility of combining in the same apparatus an EIT imager device. EIT imaging is proposed as a tool to determine the underlying tissue structure and composition of the tissue and changes in such tissue, for example to identify tumours.

Document WO 2010126827 A2 discloses methods using impedance spectroscopy for determining the concentration of blood analytes and in particular glucose. In some embodiments, a 2D or 3D EIT image is used to measure the impedance at the fingers. EIT images are used to separate uninteresting zones with little blood content such as the skin and bones from regions with higher blood irrigation, comprising veins, arteries and capillaries, which are better for determining blood analyte concentrations. Applying the electric impedance analysis in selected tissue regions with high irrigation constitutes an improvement with respect to the approaches previously disclosed. Nevertheless, a need subsists to further reduce the impact of tissue heterogeneity in the measurement, as well as the influence of temperature variations and other environmental factors.

### Summary

The present disclosure concerns a method for determining the concentration of a blood analyte of a user, the method comprising:
providing an apparatus configured to acquire EIT images within a measurement region of the thorax of the user, each EIT image comprising a plurality of EIT pixels, each of said EIT pixels comprising at least a first impedance value corresponding to a first measurement frequency, and a second impedance value corresponding to a second measurement frequency;
using the apparatus to measure a time-sequence of EIT images of said measurement region during a predetermined measuring time covering at least one heart cycle;
determining a region of interest (ROI) within the measurement region, said ROI comprising at least a section of the lungs; and
calculating the blood analyte concentration based on the impedance values of the EIT pixels within said ROI, from said time-sequence of EIT images.

The method allows for non-invasive measurement of a blood analyte in an individual which is less sensitive to tissue heterogeneity, temperature variations and environmental factors than the methods of the prior art.

More advantages of the method will become apparent in the following description.

### Brief description

Exemplar embodiments of the invention are disclosed in the description and illustrated by the drawings in which:
Fig. 1 schematically illustrates an apparatus for measuring a concentration of a blood analyte of a user, according to an embodiment;
Fig. 2 illustrates a method for determining the concentration of a blood analyte of a user using the apparatus;
Fig. 3 schematically illustrates the frequency dependence of an impedance-related value Z of a biological sample, for different concentrations of a blood analyte;
Fig. 4 shows a graphic representation of EIT images from a user's chest, according to an embodiment;
Fig. 5 and Fig. 6 show graphically different aspects of a time-sequence from a concentration parameter according to an embodiment;

### Detailed description

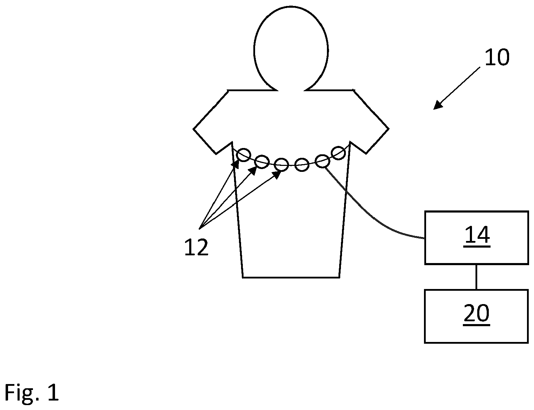

Fig. 1 schematically illustrates an apparatus 10 configured to measure electrical impedance tomography (EIT) images within a measurement region corresponding to the thorax of a user, and to calculate a concentration of a blood analyte of a user, according to an embodiment. The apparatus 10 comprises a plurality of electrodes 12 configured to be attached around the user's thorax, in contact with the skin. The apparatus 10 further comprises a control module 14 configured to control the electrodes 12, such as to inject an alternating current (current signal) at two or several frequencies through at least one of the electrodes 12 and synchronously measuring a voltage signal in the other electrodes 12. In one aspect, the plurality of electrodes 12 can comprise a first set of electrodes and a second set of electrodes. The control module 14 can be configured to control the electrodes 12 such as to inject an alternating current in the first set of electrodes and to measure the voltage in the second set of electrodes. For example, the plurality of electrodes 12 can comprise electrode pairs comprising one electrode for injecting the current and another electrode for measuring the voltage.

An EIT image can be obtained from the measured voltages from the electrodes 12, by processing methods well known to the skilled person. In general, an EIT image comprises an array of pixels mapping the measurement region in this case at least a section of the user's thorax. By "section" it should be understood a planar, a bi-dimensional cross-section, or a three-dimensional object (3D-imaging).

Each pixel within an EIT image is thus associated with a spatial location and defined by at least one impedance-related value Z. Said impedance-related value Z is most often a conductivity value (typically measured in Ω⁻¹m⁻¹), but it could also be a resistivity value (typically measured in Ω m), or a phase parameter (typically measured in rad) indicative of the phase relation between the injected current signal and the measured voltage signal calculated at the position of the pixel. Moreover, an impedance-related value Z is not necessarily an absolute value, but can be calculated relative to a reference value, for example, as an instant deviation. The reference value could be for example, the mean value of impedance averaged across all the EIT pixels, or a subset thereof. The reference value could also be the mean value of impedance measured locally at a given pixel, averaged across a defined measurement period. For the sake of simplicity, in the following we will refer to any such impedance-related value Z as, shortly, an impedance value Z.

In an embodiment, the impedance values Z of the pixels in each EIT image is determined for at least two different frequencies f1, f2. For this, the control module 14 can be configured to inject at regular intervals, a current signal at a first frequency f1, and a current signal at a second frequency f2. More conveniently, the control module 14 can be configured to inject simultaneously a current signal comprising two distinct frequency components f1, f2, which can be separated by demodulation of the measured voltage signals to calculate the frequency-dependent impedance values Z[f1], Z[f2]. This approach is more convenient because both frequency components are measured at the same time, and there is not necessarily a dead-time in the measurement of one or the other component.

In general, it may be advantageous performing the measurement with more than two frequencies. Several frequency components can be measured as explained above. There is no fundamental limit to the number of frequencies that can be measured simultaneously, but practical aspects such as the computational effort for the treatment of multi-frequency data, or signal-to-noise limitations for the demodulation of signals with a complex spectral composition will result in a compromise regarding the number of measured frequencies.

The interest of performing multi-frequency measurements is that they provide further insight into features of the impedance spectrum of the observed tissues. An unreferenced single-frequency impedance value of a complex material such as a living organism depends on too many factors and will hardly ever constitute a good indicator of the concentration of a particular analyte in the blood. In contrast, a known feature of the impedance spectrum related with the concentration of that particular analyte can be a good indicator, which can be determined from the ratio (or difference) of the impedance values of the sample at different frequencies Z[f1], Z[f2], or with the average of them.

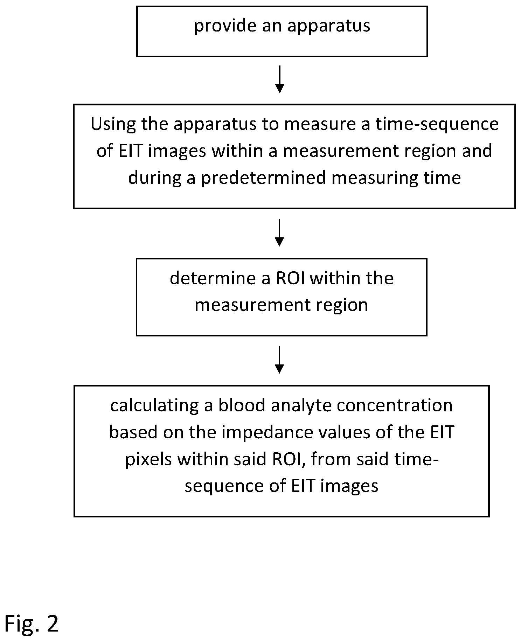

According to an embodiment illustrated in Fig. 2, a method for determining the concentration of a blood analyte of a user, comprises the steps of:
providing the apparatus 10 and using the apparatus 10 to measure a time-sequence of EIT images of said measurement region during a predetermined measuring time covering at least one heart cycle;
determining a region of interest (ROI) within the measurement region, said ROI comprising at least a section of the lungs; and
calculating the blood analyte concentration based on the impedance values of the EIT pixels within said ROI, from said time-sequence of EIT images.

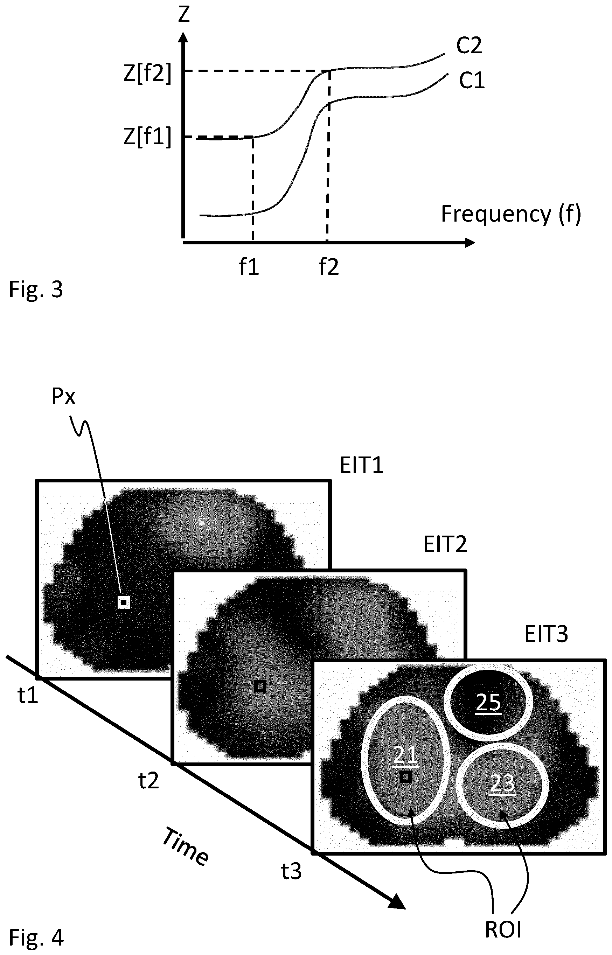

Fig. 3 shows a qualitative representation of the frequencydependency of a measured impedance value Z from a blood-perfused tissue for two different blood analyte concentrations C1, C2, of glucose as an example of analyte. While the absolute value of the measured impedance Z at a given frequency is determined mainly by the tissue constitution and by the measuring interface, the ratio R between the impedance values measured at the first frequency f1 and the second frequency f2 can be a good indicator of the glucose concentration in the blood-perfused tissue.

The ratio R defined in the example above: R = Z[f1] / Z[f2], is a simple example of extraction of an analyte-concentration-related parameter R from multifrequency impedance data. Other, more or less complex calculations, possibly involving more than two frequencies can also be defined and optimized depending on the specific analytes. For the sake of simplicity, in the following paragraphs we will refer to any variable which can be related to the concentration of an analyte, and which is calculated from multi-frequency impedance values Z[f1], Z[f2], as a concentration parameter R.

For example, it has been determined that the concentration of glucose in blood can be advantageously calculated from a first impedance value Z[f1] measured at a frequency equal or smaller than 50 kHz, and a second impedance value Z[f2] measured at a frequency equal or larger than 500 kHz, preferably equal or larger than 1 MHz. Advantageously, said impedance values Z[f1], Z[f2] can be admittance (conductivity) values. Advantageously, a concentration parameter R can be calculated from a ratio between said first and second impedance values Z[f1], Z[f2].

Since each pixel of each EIT image comprises at least a first impedance value Z[f1] corresponding to a first measurement frequency f1, and a second impedance value Z[f2] corresponding to a second measurement frequency f2, it is possible to associate each pixel to a concentration parameter R, relevant for the analyte whose concentration is to be measured.

An EIT image can be calculated from data acquired within a short time interval of 20 ms or even less. This enables the acquisition of a time-sequence of EIT images, acquired at rates of 50 images per second or even higher. For the methods of the present invention the rate of image acquisition should be preferably of at least 10 images per second, and more preferably of at least 50 images per second. It should be noted that a real-time calculation of the EIT images in parallel with the acquisition process is not a requisite of the present method. The data can be processed either in real-time or in a post-acquisition step, by a processor 20 (see Fig. 1) which can be either integrated in the measuring apparatus 10, or in a separate computer apparatus or cloud computer.

It is also worth mentioning that when we refer to EIT images in the present context, it does not mean that a graphical representation of said EIT images must necessarily be produced at some point in the implementation of the methods described here.

Fig. 4 shows a graphic representation of a time-sequence of EIT images measured in the measurement region. Three images are represented EIT1, EIT2, EIT3, acquired at different moments t1, t2, t3, within a heart cycle. In this graphics, the grey-scale colour of each pixel represents the conductivity of the tissue, measured at a first frequency f1. It is understood that EIT images can also be plotted based on other impedance values Z of the pixels, or based on a concentration parameter R.

A pixel Px has been singled out in the figure for the purpose of illustration. From the evolution of the conductivity through the heart cycle, three regions 21, 23, 25 of the measurement region can be identified which are represented in the image EIT3. On the left side of the image, a first region 21 corresponds to the right lung. (the measurement region (thorax) is represented according to a perspective where the observer is standing at the feet of the user with the user lying on his back). A second region 23, corresponds to the left lung, whereas a third region 25 corresponds to the heart of the patient.

For the purpose of determining the concentration of a blood analyte from the EIT data, it is more advantageous to consider the data from the regions corresponding to the lungs, 21, 23, than other parts of the image for the reasons that will be presented below. The first, second and third regions 21-23 which we identify as ROI may be advantageously selected to perform the subsequent steps of the methods presented herein, while discarding the information from other pixels of the image corresponding to less irrigated tissues.

One advantage of working specifically with the EIT data from the lungs is that, contrary to peripheric regions, there is a large pulsatility of the impedance values in the lungs, due to the massive arrival of blood waves in the lungs, which periodically alter the volume of blood in the measured tissue. By taking the difference between the higher and lower impedance values Z, or higher and lower values of a concentration parameter R across the heart cycle, it is possible to isolate the independent contribution of the blood arriving at the lungs from the background contribution of the lung tissue. Such feature is also useful for easy reconstruction of EIT images (time-differential method). This possibility of isolating the blood contribution improves the problematic of tissue heterogeneity known from the prior art.

Another advantage of focusing on a ROI comprising the lungs is that, contrary to peripheric regions, the temperature of the blood at the lungs is reliably stable since equal to the core body temperature. Also, there is no influence of environmental factors such as humidity or sweat that could affect the impedance values as it happens particularly when measuring directly at the skin. Of course, the absolute injected current signal and the measured voltage signal at the electrodes 12 in the present method will also be affected by different conditions of humidity, and this can introduce an offset in the impedance values calculated for the pixels of the ROI. However, the high pulsatility of the measured voltage signal from the lung region enables the extraction of variations which are associated with the blood impedance. Those variations are unaffected by an offset related to the physical contact of the electrodes 12 at the skin of the patient.

Another advantage of focusing on a ROI comprising the lungs is that the blood flushing in the lungs is representative of the general state of the whole organism. Indeed, this blood is a mix of the venous return arriving to the heart from all organs of the body. At other locations, the concentration of certain analytes in the blood may vary significantly depending on the metabolism of the organ that is analysed.

Last but not least, the lungs offer a large area of relatively homogenous tissue which makes less critical the spatial localization of the ROI, compared for example to attempting measuring the blood inside the section of an artery (e.g., aorta or main pulmonary artery), which is a rather small area of typically some squared millimetres and would require an extreme spatial resolution to obtain data from the circulating blood.

Advantageously, the impedance values Z at different frequencies, or a concentration parameter R from the pixels comprised within a ROI can be averaged to provide, respectively, a spatially averaged impedance value Z_{ROI} or a spatially averaged concentration parameter R_{ROI} with improved signal-to-noise ratio compared to a single-pixel value Z_{Px}, R_{Px}.

The ROI is not necessarily conformed by a unique set of adjacent points. On the contrary, the ROI may comprise sets of pixels from several areas not connected in the EIT images, for example the first and second regions 21 and 23 in Fig. 3.

Advantageously, the determination of the ROI may be based on the pulsatility (variability) of the impedance values Z or a concentration parameter R of the different pixels in the time-sequence of EIT images. The choice of the ROI may also take into account the anatomy of the thorax section to select areas which are known from their position to correspond to the lungs. Additionally, or alternatively, the ROI may be determined from the specific phase shift of the pulsatility signal from the pixels compared to an external heart-induced signal (e.g., ECG).

A step of defining a ROI may be executed by a technician operating the apparatus 10, based for example on a graphic interface showing a graphic representation of the EIT images and allowing the technician to define said ROI on the graphic interface. Alternatively, the ROI may be automatically determined by an algorithm, trained or more generally, designed to identify the pixels of high pulsatility, possibly taking also into account the anatomy of the thorax section with the expected placement of the lungs.

The ROI can be advantageously constituted only of pixels corresponding to highly perfused regions of the lungs (regions of the lungs that is highly perfused by blood, also referred to hereafter as regions comprising perfused tissues of the lungs). On the other hand, it is understood that the ROI may also contain additional pixels from areas peripheric to the highly perfused regions, or even comprise all the pixels of the EIT image. More generally, the ROI may comprise any section of the lungs. On the other hand, the ROI could also be constituted of a single pixel Px from the EIT image, as long as the section of the thorax corresponding to said pixel is comprised in a section of the lungs.

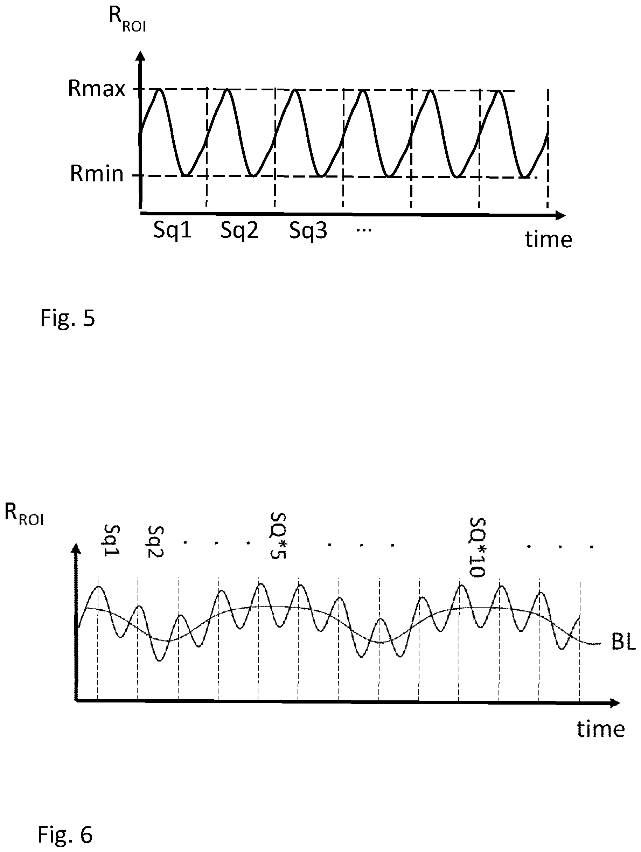

Fig. 5 shows a qualitative plot of a time-sequence obtained from the spatially averaged concentration parameter R_{ROI}. Each measurement point defining this curve is determined from one of the EIT images EIT1, EIT2, EIT3, as discussed above. The difference between the minimum and maximum values of this concentration parameter Rmax - Rmin, is representative of the contribution of the perfusing blood to the impedance measurements and can be advantageously used to calculate the concentration of the blood analyte.

Notice that, in some embodiments the maximum and minimum values of impedance values at different frequencies Z[f1], Z[f2], could be determined independently, and their difference used directly to calculate the concentration of a blood analyte without a step of calculating a time-sequence of a concentration parameter R, as plotted in Fig. 4.

In some embodiments the method can also be implemented without calculating strictly the difference between a maximum and minimum value of either the impedances or a concentration parameter. Instead of the amplitude of the oscillations, the concentration of a blood analyte can be calculated by determining the standard deviation of the of impedance values at different frequencies Z[f1], Z[f2], or any other statistical parameter, alone or in combination, related to the variability of the of impedance values Z[f1], Z[f2], averaged impedance value Z_{ROI}, concentration parameter R, or spatially averaged concentration parameter R_{ROI}. For the sake of simplicity, in the following paragraphs we will refer to any such measure of the amplitude of oscillations as a "variability" parameter.

Normally, the acquisition of the time-sequence of EIT images should be performed during approximately one second or the time it takes to capture a full heart cycle within the sequence. It is however advantageous to make a longer acquisition of at least 10 seconds, preferably 60 seconds or more, in order to capture several heart cycles which can be averaged for higher statistical significance.

As an example, in Fig. 4, the time-sequence of the spatially averaged concentration parameter R_{ROI} has been divided into a plurality of shorter sequences Sq1, Sq2, Sq3..., each of which corresponds to a complete heart cycle. These shorter sequences can be averaged together to obtain a more statistically significant averaged heart-cycle sequence, which can be used to determine the variability parameters needed to calculate the concentration of a blood analyte according to the present methods. A step of calculating an averaged heart-cycle sequence may advantageously concern the averaging of short sequences from a concentration parameter R, as in the example of Fig. 4. However, it could also be advantageously performed on a time sequence of impedance values Z, or even directly on a time sequence of a measured voltage signal from any of the electrodes 12 connected to the measuring apparatus 10.

The calculation of said averaged heart-cycle sequence can further include signal processing steps, such as: the elimination of outlier shortersequences Sqx with evidence of motion related noise or other types of artifacts; adapting the time scale of each shorter sequence Sqx to account for the heart-rate variability during the measurement; interpolating the actual measurement points into a normalized time vector for each shorter sequence Sqx, etc.

Advantageously, the division of the measured time-sequence into a plurality of shorter sequences Sq1, Sq2, Sq3, as well as other possible steps such as adapting the time scale to the instantaneous heart rate, can be performed accurately by reference to a simultaneously acquired ECG signal. An ECG signal reflects the heart cycles in a very accurate and well-known manner. Said ECG signal can be advantageously acquired by one or several of the electrodes 12 of the measuring apparatus 10 described before, or by an additional sensor synchronized with said measuring apparatus 10.

As mentioned before, the impedance values from the thorax, and particularly those corresponding to the lungs, are subject to heart-cycle variations, which advantageously contribute to the accuracy of the present methods. Superimposed to those heart cycle variations, there is an additional base-line oscillation BL related with the respiratory cycle of the patient. Indeed, as the lungs are inflated with air, their average conductivity is reduced by the presence of the non-conducting (or high impedance) air. This oscillating base-line BL is qualitatively represented in Fig. 6. Fortunately, the person skilled in signal processing will be able to separate the heart-induced variability from the base-line variability in such signal by implementing known methods including for example Fourier filtering, or Principal Component Analysis methods. An alternative to such methods could comprise perform respiration-gated ensemble averaging, i.e., to calculate the averaged heart-cycle sequence based only on those short sequences SQ*5, SQ*10 which correspond to heart cycles occurring always at the same phase of the respiratory cycle; preferably when the lungs are fully inflated or deflated, for minimal base-line variation during said selected short sequences SQ*5, SQ*10. Advantageously, it is possible to use the time-sequence of EIT images to determine the phases of the respiratory cycle. Another approach can comprise averaging all the short sequences Sqx spanning across several respiratory cycles, preferably 10 or more respiratory cycles, such as to average out the fluctuations of the respiratory baseline BL.

The step of calculating the concentration of a blood analyte from either the variability of a concentration parameter R, or directly from the variability of impedance values of different frequencies, Z[f1], Z[f2], can be advantageously performed based on a correspondence table, which can be either universally calibrated or can be preferably personalized.

The personalization of such correspondence table obviously requires a calibration phase. Said calibration phase can be advantageously performed by implementing all the steps of the present method, up to the determination of a variability parameter relevant for the analyte under study and simultaneously measuring the concentration value of the blood analyte by another previously known method. Said previously known methods may include, for instance, taking a sample from the patient's blood or from another fluid and performing a chemical, electro-chemical or spectroscopy measurement, capable of determining the concentration of the target blood analyte.

The calibration procedure may require voluntarily changing the blood analyte concentration of the patient to obtain several calibration points (for example, providing sugar-rich food to the patient to increase their glucose levels, or insulin to reduce them). However, depending on the analyte, it is also possible that only one calibration point may be enough to adapt a universal correspondence table into a personalized measurement.

A universal correspondence table for a specific analyte can be obtained advantageously from multiple calibration procedures as described above performed on a large variety of individuals.

Alternatively, the step of calculating the concentration of a blood analyte from either the variability of a concentration parameter R, or directly from the variability of impedance values of different frequencies, Z[f1], Z[f2], can be performed by a machine learning model, trained on the calibration data from a large variety of individuals, and/or from a specific patient. The input for such machine learning model can be pre-processed data, including for example variability parameters of impedance values at different frequencies, Z[f1], Z[f2], or variability values of one or several concentration parameters R. Advantageously, the machine learning model could also be trained to calculate the concentration of the blood analyte directly from the time sequence of any of these parameters, leaving the interpretation of the variability data to the artificial intelligence. Also possibly, the machine learning model could be trained to calculate the blood analyte concentration directly from the raw data extracted from the measuring apparatus 10.

The present disclosure further concerns a computer program comprising instructions for implementing the method.

Also disclosed is an apparatus 10 configured to perform the method disclosed herein.

For example, the apparatus 10, adapted for determining the concentration of a blood analyte of a user, comprises the plurality of electrodes 12 and the control module 14. The latter is configured to control the electrodes 12 to inject a current signal at two or several frequencies through at least one of the electrodes 12 and measuring a voltage signal in the other electrodes 12. The apparatus 10 is further configured to measure EIT images within a measurement region of the thorax of a user, each EIT image comprising a plurality of EIT pixels, each of said EIT pixels comprising at least a first impedance value Z[f1] corresponding to a first measurement frequency f1, and a second impedance value Z[f2] corresponding to a second measurement frequency f2. The apparatus 10 further comprising a processor 20 that is configured to determine a ROI within the measurement region, said ROI comprising at least a section of the lungs. The apparatus is further configured to calculate a blood analyte concentration based on the impedance values of the EIT pixels within said ROI, from said time-sequence of EIT images.

As mentioned above, the processor 20 can be configured to process the data either in real-time or in a post-acquisition step. The processor 20 can be integrated in the apparatus 10, or in a separate computer apparatus or cloud computer. In the two latter cases, the processor 20 can be configured to communicate with the apparatus 10.

## Claims

1. Method for determining the concentration of a blood analyte of a user, comprising:
providing an apparatus (10) configured to acquire electrical impedance tomography (EIT) images within a measurement region of the thorax of the user, each EIT image comprising a plurality of EIT pixels, each of said EIT pixels comprising at least a first impedance value (Z[f1]) corresponding to a first measurement frequency (f1), and a second impedance value (Z[f2]) corresponding to a second measurement frequency (f2);
using the apparatus (10) to measure a time-sequence of EIT images of said measurement region during a predetermined measuring time covering at least one heart cycle;
determining a region of interest (ROI) within the measurement region, said ROI comprising at least a section of the lungs; and
calculating the blood analyte concentration based on the impedance values of the EIT pixels within said ROI, from said time-sequence of EIT images.

2. Method according to claim 1,
wherein the time-sequence of EIT images is acquired at a rate of at least 10 images per second; preferably, at least 50 images per second.

3. Method according to any of the preceding claims,
wherein calculating the blood analyte concentration comprises calculating a concentration parameter (R) by using an operation involving at least two impedance values (Z[f1], Z[f2]) measured at said first measurement frequency (f1) and at said second measurement frequency (f2).

4. Method according to claim 3,
wherein said operation of calculating a concentration parameter (R) comprises calculating a ratio between said at least two impedance values (Z[f1], Z[f2]).

5. Method according to any of the preceding claims,
wherein calculating the blood analyte concentration comprises calculating a variability parameter of either an impedance value (Z), or a concentration parameter (R).

6. Method according to any of the preceding claims,
wherein the determination of the ROI is based on a variability parameter of the EIT pixels within said time-sequence of EIT images.

7. Method according to any of the preceding claims,
wherein the ROI comprises the perfused tissues of the lungs.

8. Method according to any of the preceding claims,
further comprising a step of calculating a spatially averaged value (Z_{ROI} , R_{ROI}) of either the impedance values (Z), or the concentration parameters (R), from the pixels corresponding to said ROI.

9. Method according to any of the preceding claims,
wherein the predetermined measuring time covers at least 10 seconds, preferably more than 60 seconds.

10. Method according to claim 9,
wherein the apparatus (10) comprises a plurality of electrodes (12) and a control module (14) configured to control the electrodes (12) such as to inject an current signal through at least one of the electrodes (12) and measuring a voltage signal in the other electrodes (12); and
wherein the method further comprises a step of calculating an averaged heart-cycle sequence of either a measured voltage signal of an electrode (12), an impedance value (Z), or a concentration parameter (R).

11. Method according to claim 10,
further comprising using an ECG sensor to acquire an ECG signal synchronously with the sequence of temporally discrete EIT images; and using said ECG signal as temporal reference in the step of calculating an averaged heart-cycle sequence.

12. Method according to claim 11,
wherein said ECG sensor is comprised in said apparatus (10).

13. Method according to any of the preceding claims,
wherein the first measurement frequency (f1) is equal or below 50 kHz, and the second measuring frequency (f2) is equal or above 100 kHz, preferably equal or above 1 MHz.

14. Method according to any of the preceding claims,
wherein said blood analyte is glucose.

15. Method according to any of the preceding claims,
wherein the step of calculating the blood analyte concentration comprises using a machine learning model trained on calibration data obtained from a large variety of individuals and/or calibration data from a specific user.

16. An apparatus (10) for determining the concentration of a blood analyte of a user, comprising:
a plurality of electrodes (12) and a control module (14), the control module (14) being configured to control the electrodes (12) to inject a current signal at two or several frequencies through at least one of the electrodes (12) and measuring a voltage signal in the other electrodes (12);
the apparatus (10) being further configured to measure EIT images within a measurement region of the thorax of a user, each EIT image comprising a plurality of EIT pixels, each of said EIT pixels comprising at least a first impedance value (Z[f1]) corresponding to a first measurement frequency (f1), and a second impedance value (Z[f2]) corresponding to a second measurement frequency (f2);
the apparatus (10) being further configured to execute the method according to any one of claims 1 to 15.
